# EUROPEAN PATENT APPLICATION

(11) **EP 2 055 316 A1**
(43) Date of publication of application: **06.05.2009**
(21) Application number: 08020274.0
(22) Date of filing: 09.09.2004
(51) Int. Cl.: A61K 48/00, A61K 39/21

(54) **Lentivirus vector-based approaches for generating an immune response to HIV in humans**

(30) Priority: 09.09.2003 US 501665 P
(62) Divisional of application: 04788661.9
(71) Applicant: VIRxSYS Corporation, Gaithersburg, Maryland 20877 (US)
(72) Inventor: Lu, Xiaobin, Germantown MD 20874 (US); Dropulic, Boro, Ellicott City MD 21042 (US)
(74) Representative: Roques, Sarah Elizabeth

(57) **Abstract**

The present invention relates to multiple novel approaches for the generation of an immune response in humans using lentivirus-based vector technology. The invention provides for the ability to mimic the efficacy of a live attenuated (LA) vaccine, without exposing the patient to the risk of disease as possible with some LA vaccines. The invention thus provides for the systems of complementary conditionally replicating vectors, vectors that produce replication deficient virus like particles, and multi-antigen constructs that target a virus or microbial pathogen. The use of these materials in the practice of the invention permits the generation of robust cellular and humoral responses to the antigens presented thereby.

## Description

### Technical Field

The present invention relates to multiple novel approaches for the generation of an immune response in humans using lentivirus-based vector technology. The invention provides for the ability to mimic the efficacy of a live attenuated (LA) vaccine, without exposing the patient to the risk of disease, as previously demonstrated with LA HIV vaccines for example. Moreover, the invention prevents viral escape that has resulted in the failure of previous vaccines due the extreme changeable nature of viruses such as HIV. The control against escape is provided via use of lentiviral vector-based technology to present multiple antigens, preferably as found in, or approximating, wildtype occurrences of the antigens *in vivo.* The presentation of multiple antigens provides for the generation of a diversified immune response. The lentiviral based vectors include conditionally replicating vectors as well as those that produce non-infectious vector-like particles (VLPs). While the invention is exemplified with respect to HIV, the strategies may be readily applied to the generation of immune responses against other viruses or microorganisms, including bacteria.

### Background Art

HIV infects 42 million people worldwide. In the United States (U.S.), it is estimated that over 980,000 people are infected with HIV. The mortality due to HIV/AIDS is estimated to be approximately 3 million deaths annually worldwide, and over 15,000 in the U.S. (UNAIDS Joint United Nations Programme on HIV/AIDS. AIDS epidemic updates. (2002); Centers for Disease Control and Prevention. HIV/AIDS surveillance report. 13 (2001)). Although treatment options for HIV exist, they are expensive and have a significant negative impact on the quality of life of the patient as described below. Therefore, there is a critical need for a successful HIV vaccine.

The current standard of treatment for HIV/AIDS is the highly active antiretroviral therapy (HAART). This therapy typically consists of a triple "cocktail" of a nucleoside reverse transcriptase inhibitor (NRTI), a non-nucleoside reverse transcriptase inhibitor (NNRTI) and a protease inhibitor (PI). Although these cocktails have been successful in reducing viral loads and restoring immune function, they do not represent a cure, and there are concerns regarding adverse effects associated with long-term usage of HAART. Specifically, a variety of metabolic disorders including HIV-associated lipodystrophy, central adiposity, dyslipidaemia, hyperlipidaemia, hyperglycemia and insulin resistance have been reported as resulting from HAART (Vigouroux, C. et al. Adverse metabolic disorders during highly active antiretroviral treatments (HAART) of HIV disease. Diab. Metab. 25, 385-392 (1999); Behrens G.M.N., Stoll M. & Schmidt R.E. Lipodystrophy syndrome in HIV infection. What is it, what causes it, and how can it be managed? Drug Saf. 23, 57-76 (2000); Powderly WG. Long-term exposure to lifelong therapies. J. Acq. Imm. Def. Synd. 29S, 28-40 (2002)). These reactions, combined with complex and cumbersome dosing regimes, can have an adverse impact on patient-subject adherence to therapy (Lucas G.M., Chaisson R.E., & Moore R.D. Highly active antiretroviral therapy in a large urban clinic: risk factors for virologic failure and adverse drug reactions. Ann. Int. Med 131, 81-87 (1999); Max B. & Sherer R. Management of the adverse effects of antiretroviral therapy and medication adherence. Clin. Infect. Dis. 30S, 96-116 (2000)). Furthermore, poor adherence has led to increased rate of HIV resistance, resulting in viral strains that have reduced sensitivity to the drugs (Nijuis M., Deeks S. & Boucher C. Implications of antiretroviral resistance on viral fitness. Curr. Opin. Infec. Dis. 14, 23-28 (2001); Turner B.J. Adherence to antiretroviral therapy by human immunodeficiency virus-infected patients. J. Infec. Dis. 185S, 145-151 (1993)). In fact, as many as 18.5% of newly infected HIV infected individuals in the U.S. have failed or are resistant to combination anti-retroviral drug therapy (11th International Workshop for HIV drug resistance, 2002, Rapid Report). These patients have no treatment alternatives and have very poor prognoses. This number is expected to increase since a significant rise in drug resistance between 1995 and 2000 has been documented and there is reasonable expectation that it would continue to do so. In addition, these numbers do not include individuals who are intolerant to drug therapy due to side effects (Little S.J. et al. Antiretroviral-drug resistance among patients recently infected with HIV. New Eng. J. Med. 347, 385-394. (2002)).

Historically, vaccines provide protection from virus infection by eliciting a strong antiviral neutralizing antibody response. Neutralizing antibodies recognize proteins on the virus surface and prevent binding to and infection of healthy cells. However, this approach is not effective against HIV due to the broad range of HIV subtypes and rapid mutation rate that allows HIV to escape immune responses that are not sufficiently diverse. The most successful of vaccines designed to elicit neutralizing antibody are bivalent vaccines that consist of recombinant envelope proteins derived from two different strains of HIV. VaxGen has led the field with these bivalent vaccines, but although some protective immunity is elicited, the immune response to these vaccines remains poor. Although unclear, it is thought that the reason for the poor protection of this vaccine is that it does not stimulate a robust and diverse cellular immune response in addition to the humoral (antibody-based) immune response that it generates. Researchers are also developing HIV vaccines based upon the cellular immune response. Cellular immunity is based upon a type of white blood cell called cytotoxic T-lymphocytes (CTLs), or CD8+ T lymphocytes, which kill cells that are infected with virus. This approach prevents amplification of virus in the body so that disease does not develop and the virus cannot be transmitted to another individual.

Several groups have tested vaccines based upon cellular immunity. Primarily, these studies have used recombinant carrier viruses that do not cause disease, and which carry HIV proteins as a payload. This vaccine is designed to produce the HIV protein payload carried when they are delivered into the body to elicit an immune response, but do not themselves replicate. However, there have been several problems with this approach. When using a carrier virus, the immune response reacts to the carrier virus in addition to the HIV payload, which bifurcates the response thus reducing the impact of the anti-HIV immune response. Furthermore, usually only one HIV protein can be expressed at a time due to size limitations of the carrier virus genome, thus making it easier for HIV to accumulate mutations in that single area of the genome, without significant cost to viral fitness, to escape the immune response. The most recent vaccine tested by Merck Research Laboratories and published in the scientific journal Nature in January 2002, used this approach as its vaccine strategy. Merck used a replication-incompetent adenoviral vaccine vector expressing the SIV protein gag in monkeys. However, as reported in the same journal issue, virus eventually mutated and escaped the vaccine-induced anti-HIV immune response, likely due to the fact that immunity only developed to a single HIV protein. Even though the CTL response can protect against a broader range of HIV strains, the nature of this response is that it tends to evolve against only a small region of the target HIV protein, which makes resistance likely since an evasive mutation in HIV may occur without a significant alteration in virus structure. This is especially the case when only one HIV protein is being used in vaccination.

An alternative vaccine approach is to use a live attenuated (LA), or reduced in strength, HIV vaccine. This is the approach used for many vaccines currently in use, such as the polio vaccine. LA vaccines do not cause disease in humans, but they are able to replicate and elicit a broad, well-rounded immune response consisting of both cellular immunity and a neutralizing antibody response to HIV proteins produced during infection. It is especially important that a diverse immune response be mounted against HIV since the high mutation rate of the virus during infection changes how the virus looks to the immune system thus contributing to immune evasion. A LA vaccine presents these diverse variables to the immune system thereby avoiding this problem. However, the analogous animal model of HIV, simian immunodeficiency virus (SIV) infection in monkeys, has shown that LA SIV vaccines cause disease in juvenile and neonatal monkeys. Juvenile monkeys have an immature immune response that likely facilitated the attenuated virus becoming pathogenic in these animals (Wyand S., Manson K., Montefiori D.C., Lifson J.D., Johnson, R. P, and Desrosiers R.C. Protection by live, attenuated simian immunodeficiency virus against heterologous challenge. J. Virol. 73, 8356-8363. (1999)). Interestingly, the break through virus in this vaccine did not result from viral revertants, but rather further deleted viruses that replicated faster *in vivo*. This data suggests that a LA approach is not suitable for use in humans since immunization with a LA HIV vaccine in humans with a compromised immune response and in particular children with an immature immune system could also result in disease, as seen with the studies with LA SIV vaccines in monkeys.

Perhaps more importantly, the risk of using an attenuated HIV vaccine is represented by a group of people who were inadvertently infected with an attenuated (delta-Net) strain of HIV through a blood infusion from an infected individual (Deacon N.J. et al. Genomic structure of an attenuated quasi species of HIV-1 from a blood transfusion donor and recipients. Science. 270, 988-991 (1995); Learmont J.C. et al. Immunologic and virologic status after 14 to 18 years of infection with an attenuated strain of HIV-1. A report from the Sydney Blood Bank Cohort. N. Engl. J. Med. 340, 1715-1722. (1999)). In results that appear similar to those of the LA SIV in juvenile monkeys, half of those infected with the delta-Nef virus subsequently experienced a decline in their CD4+ T lymphocytes, which is the primary indicator of progression to AIDS. The unfortunate reality of a LA HIV vaccine that is demonstrated by these individuals is that unlike most LA vaccines, LA HIV quickly establishes latency, or infection of CD4+ T lymphocyte reservoir in the host, which then can cause disease at any time in the future.

The difficulty in generating a successful HIV/AIDS vaccine is underscored by the fact that patients do develop a strong anti-HIV response that leads to chronic hyper-activation of the immune system. However, instead of leading to HIV control, immune activation results in dysregulation of cytokines, and allows HIV to replicate at high levels in the lymphoid tissue during the early stage of infection that eventually leads to destruction of the lymph nodes and the thymus at the late stage. Ironically, activation of CD4+ T lymphocytes (central to the development of an adaptive immune response) simply increases HIV production in infected cells while healthy activated cells provide additional fuel for virus propagation.

Control of HIV *in vivo* and AIDS long term non-progressors (LTNPs) has been linked to the CD8+ T lymphocyte cytotoxic response and not to the antibody response (Gea-Banacloche, J. C. et al. Maintenance of large numbers of virus-specific CD8+ T cells in HIV-infected progressors and long-term nonprogressors. J. Immunol. 165, 1082-1092. (2000)). It is thought that weakening of this response is a result of loss of CD4+ T lymphocyte help as the lymphocytes are killed by HIV mediated death, HIV associated apoptosis, or CTL-mediated killing of HIV-infected CD4+ T lymphocytes. The presence of LTNPs among HIV patients indicates that host and viral factors influence the pathogenesis of HIV/AIDS. Specifically, persons deficient in the chemokine receptor CCR5 fail to become infected with HIV. The patients discussed earlier who were infected with naturally acquired attenuated HIV viral genomes (nef-deleted) also exhibited delayed progression to AIDS.

Although ultimately inadequate at this point, immunity does affect HIV replication and progression to AIDS since vaccines based upon attenuated virus, defective viral particles, or a prime / boost strategy using DNA and antigen-expressing vectors respectively, were capable of delaying disease progression and containing virus replication in the short term (Amara R.R. et al. Control of a mucosal challenge and prevention of AIDS by a multiprotein DNA/MVA vaccine. Science. 292, 69-74. (2001); Deacon et al, *supra*, 2002, Wyand et al., *supra*, 1999). African Green monkeys and Sootey Mangabey monkeys do not develop AIDS after infection with SIV, nor do they exhibit any lymph node or thymic destruction, thus suggesting that a balance between the virus and the host is possible.

To date, no vaccine has yet been successful in preventing the HIV infection rate, or in delaying the onset of AIDS. It has become clear that conventional vaccine strategies, which have been successful against other viruses, bacteria, and even cancer, are not applicable for HIV. This is a result of the unusually high mutation rate of HIV, even in comparison to other retroviruses, and the fact that the target of the virus is the immune system itself, enabling a kamikaze-like quality of the immune system that may be as destructive as the virus itself. Ideally, the best vaccine approach would be one that prevents *de novo* HIV infection. However, an additional approach would be to develop a vaccine that may be used in HIV-infected individuals and / or naïve individuals to boost immunity to a protective level that suppresses virus replication *in vivo* and prevents the onset of AIDS without additional therapeutic treatment, thus making HIV "harmless". To achieve these goals, novel strategies distinct from those previously used in vaccine strategies, which boost immunity while suppressing HIV replication, must be employed.

New diseases are continually emerging at least each decade, such as HIV in the 1980s, EBOLA in the 1990s, and SARS in the 2000s. Each time a new disease emerges, significant amounts of research resources are devoted to developing a protective vaccine for the disease, as each disease requires different types of vaccines to effect protection. This is the case because some diseases are controlled by humoral responses, some by cellular responses, and some require both. With each new disease, the risk of a live attenuated vaccine must be assessed, as well as the efficacy of a killed virus or recombinant protein vaccine. However, a vector-based approach to vaccination that elicits diversified cellular and humoral immunity, would allow a single vaccine approach for all diseases that may be re-engineered according to the genetic structure of each emerging threat.

Citation of the above documents is not intended as an admission that any of the foregoing is pertinent prior art. All statements as to the date or representation as to the contents of these documents is based on the information available to the applicant and does not constitute any admission as to the correctness of the dates or contents of these documents.

### Disclosure of the Invention

The present invention provides multiple novel approaches for the generation of an immune response, preferably against viruses such as HIV, in humans using lentivirus-based vector technology. Without exposing a treated subject to the risk of disease as previously observed with live attenuated (LA) vaccines, the invention provides compositions and methods that are able to mimic or reproduce the efficacy of a LA vaccine. Moreover, the invention addresses the phenomenon of high mutation rates that result in viral escape from the effects of previous vaccines.

Thus in a first aspect, the invention provides for lentiviral vector-based technology that utilizes conditionally replicating virus for antigen presentation *in vivo*. This vector-based technology allows for the expression of multiple antigens to allow for the generation of a diversified immune response, although the response does not necessarily have to be against every antigen or epitope presented by the invention. All that is needed is the generation of a response to one or more antigens expressed by the use of the invention. Preferably, the response is cellular and humoral in nature, although the occurrence of either response may occur in the practice of the invention. Even more preferred are response(s) that are protective against subsequent challenge with the antigen(s) or pathogens that present the antigen(s).

A vector based approach offers several advantages. These include concurrent presentation of multiple antigens; no (or reduced) bifurcation of the immune response between a heterologous virus carrier and the immunogens; mimicking of wild-type virus replication in the context of conditional vector replication; and extended period of antigen production. The invention thus provides for the use of a system of multiple (two or more) complementary, but individually replication-defective vectors, or conditionally replicating viral vectors such as conditionally replicating HIV vectors (crHIVs). This design provides a vaccine vector system that is safer than live attenuated (LA) virus and yet more potent than single replication defective vectors.

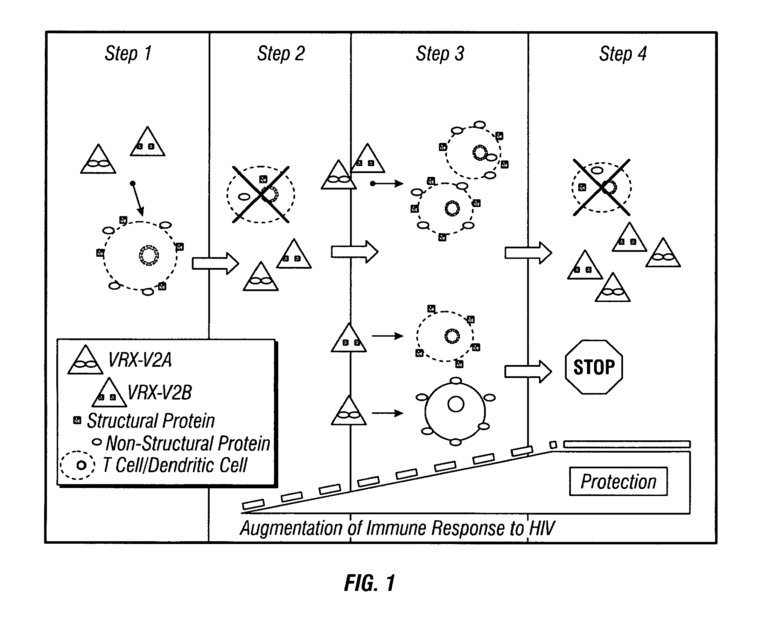

As a non-limiting example, two complementary, replication-defective HIV-1 vectors can provide limited replication and packaging of both vectors cells based on observations made by the present inventors using more than one vector, one of which expresses a VSV-G envelope protein to complement replication of another vector. The replication of an individual crHIV is always suboptimal since the system requires co-infection of the very same cells with the necessary complementary vectors in order to produce viable virus particles. When cells are infected with these vectors, multiple HIV antigens are produced to elicit robust humoral and cellular immune responses similar to that seen with vaccination using a LA virus vaccine. Each vector alone cannot replicate itself and so cannot spread to new cells. But if the necessary complementary vectors are present in the same cell, each of them supports the replication and packaging of the other as if the cells were infected with naturally occurring HIV. Progeny vaccine vectors subsequently infect other (neighboring) cells, thus propagating the immune response. This is represented schematically in Figure 1.

During replication by these complementary vectors, all of the HIV proteins necessary for replication and packaging of HIV to produce infectious viral particles are expressed, along with the opportunity for mutations to occur and provide some diversity in the expressed viral antigens (via the error-prone reverse transcription process, for example). This generates breadth in the resultant immune responses to the vectors, and thus to the target virus or microbial agent, so that mutation based escape of a target virus or microorganism is minimized after administration (post-vaccination) of the vectors. The mutations in the vectors are expected to mimic the mutations seen with LA virus replication, thus eliciting a diverse immune response against the mutated variants while the vectors propagate, until they are eventually cleared by the elicited immune responses. The ability of the vectors to replicate and mutate in a conditional manner thus provides an "active" vaccine that constantly provides new antigenic displays to elicit a robust and broad immune response better able to protect against pathogens, such as HIV, than any of the current alternative vaccines being tested. It should be noted, however, that this active production of variant antigens does not increase the potential for virulence because the underlying vectors are inherently made replication-defective such that more than random mutation of individual coding regions is necessary for restoration of replication competence.

There are multiple configurations of complementary vectors that may be designed based on the separation of necessary viral protein(s) among the vectors. In theory, any essential viral protein may be provided in *trans*, and will be utilized by vectors needing it for replication as part of the invention's intended (but non-limiting) therapeutic mechanism. To ensure that no replication competent vectors (RCVs) are generated by recombination between the complementing vectors, molecular designs (genetic antiviral agents) are included in at least one, optionally each, vector. Examples of such agents in each vector include, but are not limited to, targeted antisense sequences, ribozymes, and post-translational gene silencing (PTGS) directed to the other vector(s). Examples of PTGS include small interfering RNAs (siRNAs) and RNA inhibition (RNAi) as described below. As a non-limiting example, and to ensure sufficient propagation of complementing vectors in a coinfected cell, such agents may be differentially targeted in the cells. For example, one vector may express an agent that expresses in the nucleus while the agent expressed by the second vector traffics to the cytoplasm. Another non-limiting approach to prevent recombination between the two vectors without curtailing their independent propagation is to place the expression cassette in one of the two complementary vectors in a reverse orientation. Since the likelihood of flipping back to the original orientation through recombination is extremely low, or perhaps impossible, then the possibility of recombination is avoided and targeted agents may not be needed. Viral factors needed in *cis* for replication, however, will be present on all conditionally replicating vectors that are to be susceptible to packaging and proliferation.

Conditionally replicating vectors may be pseudotyped with any suitable envelope protein, including, but not limited to, the VSV-G envelope protein, a native HIV or HTLV envelope, or any molecule that targets CD4+ T lymphocytes and/or macrophages or dendritic cells. The pseudotyped particles may contain at least one copy of each of the vectors necessary to complement replication of them all. Alternatively, the particles may not contain at least one copy of each of the vectors necessary to complement replication of them all, but instead contain less than all of the vectors. A combination of particles providing all of the necessary vectors may still be introduced into cells via the use of particles that can multiply infected the intended cells to provide the necessary combination of particles. As a non-limiting example in the case of two complementary vectors, each can be separately packaged into particles which are then contacted with target cells for infection under conditions where the cells would be multiply infected with the particles such that some, many, or all of the cells would be infected with at least one copy of each vector.

The conditionally replicating vectors may be used to transduce highly concentrated target cells *ex vivo* using autologous cell transplantation. Alternatively, complementing vectors may used *in vivo*, such as by injection made intramuscularly, subdermally, systemically, or in an area targeted for direct drainage into the lymphatic system. Boosters may consist of simple DNA vaccinations given intramuscularly, or subdermally using the DNA of one or more of the conditionally replicating vectors. Alternative boosters by use of other genetic (vector) or proteinaceous vehicles (e.g. vaccines) may also be used to provide complementary factors in *trans*. As a non-limiting example, if a two vector system is used wherein the first vector provides functional Tat protein to permit the replication of the second vector, delivery of Tat protein, or other vector capable of expressing Tat protein, may be used to activate replication of the second vector.

The above complementary vector system may also be modified to produce non-infectious virus-like particles (VLPs). Preferably, the VLPs are lentivirus-like particles, but they may be a hybrid of viral and cellular components that make up the particles, such as, but not limited to the case of particles composed of HIV-1 proteins except for the use of an HIV-2 or other modified or heterologous env protein. The simplest modification is to omit viral factors necessary in *cis* from one or more of the vectors. Thus while a cell containing all the vectors of the system would produce viral particles using all of the necessary (and optionally non-essential) replication and packaging viral proteins, at least one of the vectors would not be capable of being packaged into the particles. This permits control over the propagation, from one cell to another, of the ability to produce VLPs.

Alternatively, the coding sequence of one or more viral factors necessary in *trans*, but present upon initial infection with a viral particle, may be mutated or omitted such that only one round of replication and packaging may occur. A non-limiting example in the case of HIV is a mutation in, or deletion (in whole or in part) of, the *pol* gene that prevents expression of reverse transcriptase and/or protease activity. Thus a vector containing such a mutation or deletion may be packaged *in vitro* with a helper/packaging vector which provides reverse transcriptase and/or protease activity in *trans*, which is also incorporated into the resultant particle to permit one round of replication and packaging after introduction into a susceptible cell with the necessary complementary vectors.

In a second aspect, the invention provides for the production of non-infectious vector-like particles (VLPs) without the use of two or more complementary conditionally replicating vectors. In this aspect, lentiviral vector-based production of antigens simultaneously stimulate cellular and humoral immunity for maximum response against a virus or microbial agent, such as, but not limited to, HIV. In the non-limiting example of HIV, this approach generates a robust and protective immunity against HIV by administration of (vaccination with) a vector which maximizes the presentation of the HIV antigen spectrum. An HIV-based vector encoding replication defective-virus like particles (RD-VLPs) is designed to generate both humoral and cell mediated immune responses. This (vaccine) vector may be viewed as "Toti-VacHIV" for its total (or nearly total) presentation of the HIV antigens and ability to stimulate both antibody and cell mediated immune responses. Therefore, the protection provided by this vector is expected to be more comprehensive than traditional single modality (single antigen) vaccines or vaccines that present limited numbers of antigens. The invention, however, contemplates the use of "nearly total" vectors that present less than all possible antigens, and antigenic epitopes, of HIV or any other targeted virus or microorganism.

Toti-VacHIV is designed to express epitopes from each viral protein as they would be processed by cells *in vivo.* Preferably, Toti-VacHIV is a lentiviral based vector comprising both 5' and 3' LTR elements and a heterologous (to HIV or other lentiviral vector) constitutively active promoter that directs expression of HIV encoded gene products after introduction into a host or target cell. This may be following conversion of the vector into a DNA form via reverse transcriptase activity. The choice of a constitutively active promoter may be any preferred by the skilled person, including, but not limited to, that derived from simian cytomegalovirus (S-CMV-P). Alternatively, a heterologous inducible promoter may be used in place of a constitutively active promoter.

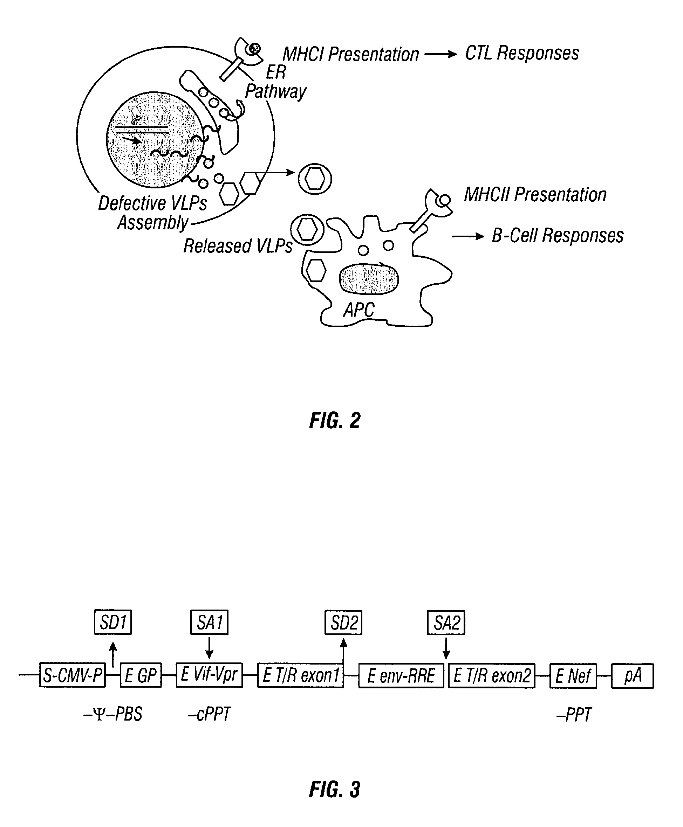

The epitopes, in the case of HIV and other lentiviruses and some retroviruses, may include, but are not limited to, those from the gag-pol, vif, vpr, and env regions, derived from the unspliced or partially spliced messenger RNA, as well as the epitopes from the tat, rev, and nef regions, which are derived from the multiply spliced mRNA. *De novo* synthesis of these antigens in cells containing the vector, such as vaccinated antigen presenting cells, are directed to the MHC class I pathway for generation of cell mediated immune responses. One beneficial feature of this vector, as well as the complementary vectors discussed above for the production of VLPs, is the ability to make completely defective VLPs by deleting of some of the functionally vital regions of gag-pol and env structural genes such that the vector(s) retain the ability to result in assembly of the VLPs in cells. The VLPs that are released from cells containing the vector(s) will be able to induce antibody responses. An advantage to the VLPs is that some of the antibodies generated will be directed to the viral conformational epitopes as found on the surface of viral particles and thus effect virus neutralization (see Figure 2).

To construct a defective virus-like particle vector system, sizeable mutations may be introduced in critical regions of viral genes and elements (e.g. *cis*-acting elements). Multiple deletions in the viral sequence will dramatically reduce the possibility of reversion of the vector to a replication competent virus. As a non-limiting example, the cis-acting packaging signal (ψ), primer binding site (PBS), the central polypurine tract (cPPT) and/or the polypurine tract (PPT) are all removed from the vector to prevent it from being packaged and/or reverse transcribed. In addition, functional regions encoding reverse transcriptase (RT) and/or integrase (IN) in the pol region, and envelope (env) structural genes, may be deleted to wholly ensure defective replication of the vector. In this process, the gag function for VLP assembly is preserved to ensure the production of the defective particles for generation of an antibody response as described above (see Figure 3). Alternatively, portions of the RT, IN and/or env encoding sequences may be retained if immune responses directed at the epitopes encoded by those portions are desired. Preferably, such portions do not encode the normal functions of RT, IN or env beyond those necessary to produce VLPs.

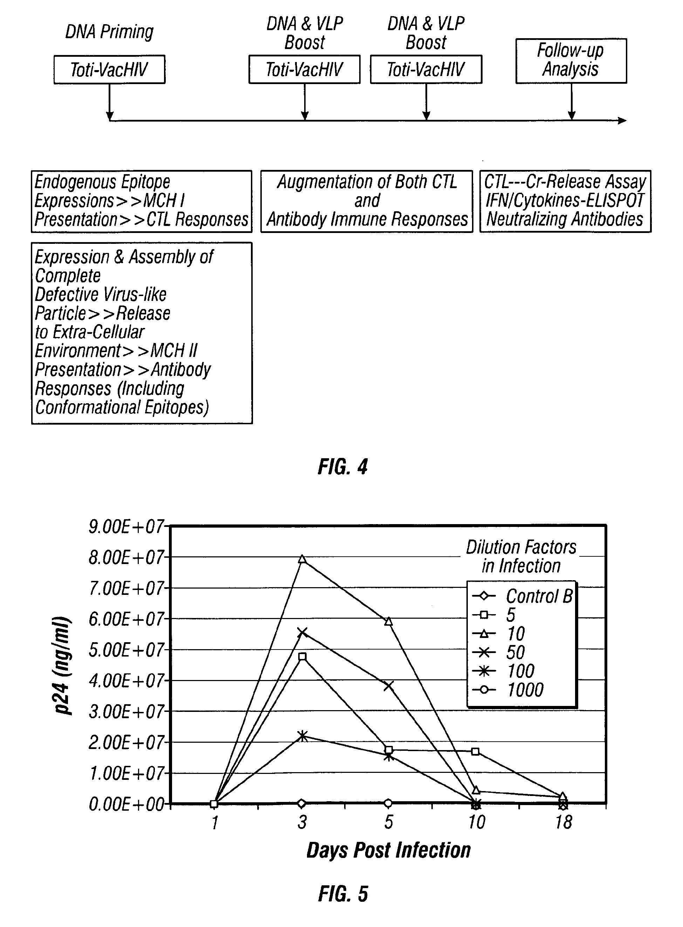

Non-limiting methods for delivery of the vaccine vector to a subject or patient include, but are not limited to, the intramuscular, subdermal, or systemic route using naked DNA in the presence of an adjuvant, followed with booster injections using Toti-VacHIV DNA and VLPs generated in culture (see Figure 4). Alternatively, the vaccine may be given via *ex vivo* transduction of target cells, most preferably lymphocytes, and/or macrophages or dendritic cells (or other antigen presenting cells) using vectors packaged in a lentiviral packaging system that pseudotypes the vector with an appropriate envelope protein, such as, but not limited to, the G protein from vesicular stomatitis virus (VSV). In the latter case, cis-acting elements, such as the ψ, PBS, and PPT elements in HIV would be retained in the vector.

Given the extensive deletions in essential regions of viral genes and *cis*-acting elements, the possibility of vector reversion to a replication-competent form of HIV can be quite reasonably made non-existent or nearly so. Thus, this vaccine approach offers a qualitative improvement from the use of a LA HIV virus, which has been shown to be pathogenic in monkeys and humans.

In a third aspect of the invention, the use of multi-antigen expressing vectors is provided. Person to person variation in antigen recognition results primarily from the polymorphism present in the peptide binding site of the major histocompatibility complex (MHC) class I and II molecules, which function to present foreign antigens to T and B cells, respectively, to result in generation and evolution of the immune response to the presented antigens. The specific peptide presented will differ depending upon the conformation of the peptide binding site in the MHC molecule. Therefore, a vaccine applicable across a broad human population will preferably use several peptide antigens to ensure effective stimulation among most or all subjects treated. In addition, it has been demonstrated that in the case of HIV infections, long term non-progressors (LTNPs) have a significantly more diverse anti-HIV CD8+ T lymphocyte response than typical HIV patients, thus underscoring the importance of generating a diverse immunity against HIV/AIDS in any vaccine approach.

The epitopes that are recognized by a more developed host immune system, such,as that of a mammal or primate, can be divided into 3 major classes according to the responding cells: cytotoxic T lymphocytes (CTL), helper CD4+ T lymphocytes (CD4), and B cells. The B cell epitopes may be further categorized into conformational and linear epitopes depending upon whether the epitope is recognized as a three dimensional native structure, or as a denatured linear entity. A vast number of immune dominant epitopes have been experimentally defined in conjunction with their associated MHC genes, and may be easily expressed as part of a multi-antigen presentation by a vector of the invention.

The invention provides for any of the vectors disclosed herein to contain a comprehensive spectrum of epitopes representing different HLA types. The invention can also be used to functionally determine epitopes for various disease-causing infectious agents, including HIV-1 and HIV-2, in the context of MHC restriction. The advantage of this approach over the existing synthetic peptide is that native and naturally, rather than artificially, processed epitopes are selected. While a disadvantage of previous peptide vaccines is the difficulty to produce synthetic molecules mimicking conformational epitopes (immunological determinants in the native protein which are formed by amino acid residues brought together as part of protein folding), the vectors of the present invention are designed to produce proteins and antigens in a more native context. Multiple members of the identified antigens and epitopes may then be combined for expression in the vectors of the invention.

Such a multi-antigen vector contains one or more sequences encoding many conserved dominant epitopes for stimulation of (and recognition by) CTL, CD4+ and/or B cells linked together as one or more polypeptides. Preferably inserted between the epitopes are conserved peptide antigen processing sequences. The epitopes are designed to cover each viral protein for development of a multivalent vaccine designed to reduce the likelihood of virus mutants capable of escaping the ensuing immune response. Stated differently, the epitopes are preferably those conserved in many or all strains of a virus or other pathogen. The identification or determination of various epitopes as potentiating CTL, CD4+ and/or B cell responses, particularly strong and protective responses, may be by any method known in the art. Such epitopes, or appropriately representative members thereof, are preferably used in the practice of the invention to stimulate a strong and protective cellular and humoral immune response in the subject.

The above strategies maybe used independently, or in combination with each other and/or with other vaccine strategies known to the skilled person, for administration to a subject in need thereof. Such subjects include individuals who are already infected with a virus or microbial agent, such as HIV, as well as individuals at risk for such infections. Administration may be by any means known in the art, including, but not limited to contacting one or more cells of said subject with the compositions of the invention. The disclosed strategies may be easily reengineered to generate immune responses, and protective states of vaccination, against many other viral or bacterial infections, or cancer. Preferred subjects for the practice of the invention are humans, although the invention may be adapted for use in other organisms, particularly mammals and primates.

Therefore, the invention provides compositions such as the above nucleic acid constructs and vectors, viral particle encapsulated forms thereof, formulations for their use, and methods of their use to generate immune responses, and protective states of vaccination, against many other viral or bacterial infections, or cancer. Additionally, the invention may be embodied in the form of kits comprising components of the invention for the practice of methods disclosed herein.

### Brief Description of the Drawings

Figure 1 shows a schematic of one embodiment as representation of the strategy of the present invention using a system of complementary, replication deficient vectors. *Step 1:* intravenous delivery of vectors to T cells; first round of infection in cells. *Step* 2: production of 1^{st} round of vector; co-packaged vector suppressed by antisense safety feature. *Step 3*: second round of infection in T cells; co-infection yields 2^{nd} round of vector production. *Step 4*: propagation of vector infection in cells until immune-mediated clearance.

Figure 2 shows a schematic of one embodiment as representation of the strategy of the present invention using a viral like particles (VLPs).

Figure 3 shows one embodiment of the invention comprising the genome of a replication defective HIV vector to produce VLPs. Features: All epitope peptides are defective by sizable deletions to prevent revertants. Epitopes are expressed from their original configuration in the viral genome. E GP is defective in RT and IN but able to assemble budding-able virus-like particles. E vif-Vpr contains conservative epitopes. E tat, delete the essential R-domain for TAR which is not immunogenic. E env, delete the protease cleavage site between gp120 and gp41. E Nef contains deletions in RR, Myr, NBP-1, β-COP binding sites. Rev is the only functional viral protein in this system. Defective cis-acting elements: -TAR, -Ψ, -PBS, -cPPT, -PPT. Figure 3 depicts a complete replication-defective viral DNA vector with the following features: presentation of many epitopes with a single vector and antigen presentation via MHCI for CTL (endogenous epitope expression) and MHCII for antibody (virus- like particles released from vaccinated cells, epitopes can be in their native conformation).

Figure 4 shows one embodiment of the invention comprising the use of a replication defective HIV vector and VLPs to product an immune response.

Figure 5 shows the propagation of a system of complementary, replication deficient HIV-based Vectors in Cell Culture.

### Modes of Carrying Out the Invention

The invention is based upon the use of lentivirus derived vectors. A lentiviral vector minimally contains LTRs from a lentivirus and optionally packaging sequences in the 5' leader and gag encoding sequences of a lentivirus. The vector may also optionally contain the RRE element to facilitate nuclear export of vector RNA in a Rev dependent manner.

Each of the aspects of the invention described above and herein is designed to maximize the likelihood of that the immune response generated by their use will contain the diversity of an incoming pathogen, such as the HIV virus, by generating (1) a multivalent response to viral antigens that mimics the diversity among naturally mutating HIV; (2) a broad breadth of immune stimulation; and (3) a balanced humoral and cellular immune response. It is worthy to note that in a TotiVacHIV system, the native conformations of viral proteins are preserved in the context of mature virus particles, which may provide an advantage for production of neutralizing antibodies.

Quite naturally these aspects of the invention may be combined to mediate the best protective results. As a non-limiting example, a patient may first be immunized with a multi-antigen vector followed by a system of complementary, conditionally replicating vectors to facilitate diversification of the response. Alternatively, these vaccine approaches may be combined with previously tested vaccines known to the skilled person, such as a LA vaccine, a killed vaccine, or a single protein (or other recombinant) vaccine. Specifically, and as a non-limiting example, a patient may be immunized first with Toti-VacHIV to prime immunity so that he/she may be subsequently vaccinated with live attenuated HIV for development of a powerful and diverse immune response without disease.

With respect to a system of complementary, conditionally replicating vectors, Figure 1 illustrates an embodiment of the invention in which two complementary, conditionally replicating vectors, VRX-V2A and VRX-V2B, are used to generate an immune response by stimulating T cells or dendritic cells upon introduction. As shown in Figure 1, each vector alone cannot propagate (one encodes the structural proteins needed in *trans* while the other encodes the non-structural proteins needed in *trans*), but during co-infection of T lymphocytes they support the replication of one another.

Infection of susceptible mammalian cells with the vaccine vectors results in expression of HIV proteins and subsequent stimulation of immunity. During infection by only one vector in a cell, the HIV proteins encoded by the vector are still expressed, but no progeny vector is produced. Methods of introducing vectors into cells are known in the art and may be used in the practice of the invention. As a non-limiting example, the methods include those disclosed in allowed U.S. Patent Application 09/653,088, filed August 31, 2000, which is hereby incorporated by reference as if fully set forth.

When two vectors are co-packaged together, the presence, in each vector, of an antisense genetic element targeted to the opposite vector suppresses and/or prevents recombination to form a replication competent HIV vector. In addition to antisense based genetic elements, the invention may be practiced with ribozymes or sequences to generate polynucleotides for post-transcriptional gene silencing (PTGS). The use of ribozymes to inhibit gene expression and virus replication is described in U.S. patent 6,410,257 via use of a conditionally replicating vector for other purposes. PTGS is mediated by the presence of a homologous double stranded RNA (dsRNA) which leads to the rapid degradation of a targeted RNA. One form of PTGS is RNA interference (RNAi) mediated by the directed introduction of dsRNA. Another form is via the use of small interfering RNAs (siRNAs) of less than about 30 nucleotides in double or single stranded form that induce PTGS in cells. A single stranded siRNA is believed to be part of an RNA-induced silencing complex (RISC) to guide the complex to a homologous mRNA target for cleavage and degradation. siRNAs induce a pathway of gene-specific degradation of target mRNA transcripts. siRNAs may be expressed in via the use of a dual expression cassette encoding complementary strands of RNA, or as a hairpin molecule.

Steps 3 and 4 of Figure 1 illustrate further rounds of infection and propagation of vaccine vectors, which continues until immune-mediated clearance occurs, commensurate with protective immunity mediated by both cellular and humoral responses.

Of course the complementary vectors disclosed herein may be modified to express, and thus generate immune responses, against non-HIV proteins, such as proteins from other viruses or microorganisms. Non-limiting examples of such other viruses include other lentiviruses (including HIV-1, HIV-2, EIAV, VMV, CAEV, BIV, FIV, and SIV), retroviruses, and other viruses with envelope glycoproteins, including, but not limited to, togaviruses, rhabdoviruses, paramyxoviruses, herpesviruses, orthomyxoviruses and coronaviruses. When a heterologous envelope protein is to be encoded and expressed by a vector, it is preferably one that is capable of pseudotyping the vector. Non-limiting examples of suitable envelope proteins for pseudotyping include the HIV-1, HIV-2, or MMLV envelope protein; the G protein from Vesicular Stomatitis Virus (VSV), Mokola virus, or rabies virus; GaLV; Alphavirus E1/2 glycoprotein; the envelope protein from human T cell leukemia virus (HTLV); RD114, an env protein from feline endogenous virus; or the glycoproteins from other lentiviruses or retroviruses such as gp90 from equine infectious anemia virus (EIAV) or the surface glycoprotein of bovine immunodeficiency virus (BIV). Sequences encoding an envelope protein from the following viral families may also be used: Piconaviridae, Tongaviridae, Coronaviridae, Rhabdoviridae, paramyxoviridar, Orthomixoviridae, Bunyaviridae, Arenaviridae, Paroviridae, Poxviridae, hepadnaviridae, and herpes viruses. Alternatively, hybrid envelope proteins comprising portions of more than one envelope protein may be encoded and expressed by vectors of the invention.

Further methods for the expression of viral envelope proteins are encompassed by the invention. In one sense, they may be considered envelope protein replacement strategies and are particularly attractive because of the variability of env proteins among viruses, especially HIV. The AIDS Research and Reference Reagent Program of the U.S. Department of Health and Human Services, National Institutes of Health (National Institute of Allergy and Infectious Diseases, 6003 Executive Boulevard, Bethesda, Md. 20892) makes available the sequences of many variant env genes. The replacement of the env encoding sequences in a system of HIV or lentiviral vectors with a variant gene from another HIV strain or isolate better tailors the resulting vectors to generate an immune response. Moreover, they provide a different starting point for mutations in the env sequences based on the inherent mutation rate seen with lentiviral viruses such as HIV. These alternative vectors may be used to vaccinate against HIV strains which are prevalent in a particular part of the world or in a particular population or against particularly virulent strains. The vectors may also be tailored for therapeutic treatment of a particular infected patient to prevent the onset of AIDS symptoms, for example, by inclusion of sequences encoding the env protein from the particular strain of HIV infecting the patient after its isolation and identification from the patient. This is readily accomplished by cloning the env sequence and replacing the env sequence in the vector with the cloned sequence by routine methods such as the polymerase chain reaction (PCR) and other recombinant DNA techniques. In another embodiment, combination of vectors encoding a multiplicity of different Env proteins may be used in the practice of the invention. Variant env encoding sequences can also be engineered by mutagenesis and used in the practice of the invention.

In preferred embodiments of the invention, and for treatment or prevention of viral infections in a subject, the vectors are used with (and express) native envelope proteins that are expected or found in the targeted virus(es). In addition to permitting an immune response to be generated against the envelope proteins of the targeted virus(es), this approach reduces or minimizes the possibility of the targeted virus(es) being repackaged with a heterologous envelope protein that would facilitate viral spread. Additionally, the vectors of the invention may encode or contain anti-viral agents that prevent the packaging of vectors with copies of the targeted virus(es) in a viral particle. Alternatively, the vectors of the invention will contain elements and agents to reduce or minimize the likelihood of recombination between a vector of the invention and a wildtype virus. Non-limiting examples of such agents and elements are provided in U.S. Patent 6,168,953, and allowed U.S. Patent Application 09/667,893, filed September 22, 2000, both of which are hereby incorporated by reference as if fully set forth.

The vectors may also encode other viral proteins, including, but not limited to, capsid proteins from other lentiviruses or other retroviruses. Indeed, virtually any protein, structural or non-structural, of a virus or microorganism which may generate a helpful immune response may be expressed by the vectors and methods of the invention as long as they do not prevent replication and/or gene expression from the vector. These proteins may also be subject to the replacement technique described above for env encoding sequences. Moreover, and as would be clear to the skilled person, the vectors of the present invention may be based upon the genomes of other lentiviral vectors as disclosed herein. The use of other lentiviral based vectors may also be used to address situations where expression of a heterologous protein interferes with vector replication and/or gene expression in that the interference may be lessened or not present when another lentiviral genome is used. In the case of expressing coronavirus proteins, the invention also provides for the expression of proteins that provide an immune response, and/or a protective effect, against the virus that causes SARS (Severe Acute Respiratory Syndrome).

As one representative embodiment, vectors based upon HIV may be constructed with mutations of the gp160 cleavage site which block the processing of the gp160 envelope precursor to gp120 and gp41. This may reduce the "shedding" of gp120 antigen observed during propagation of HIV based particles. The gp41 portion of gp160 is a transmembrane peptide which may tether the gp120 antigen more firmly to the membrane in which gp160 is inserted. The increased retention of the gp120 portion should improve the immunogenic properties of the vectors of the invention, and may be adapted to any other antigen where inhibition of "shedding" or increased retention in membranes may be useful in generating an immune response. As another representative embodiment, the env protein may be a chimeric glycoprotein, such as one having elements from both HIV-1 and HIV-2, or having a portion from the V3 loop of the MN viral isolated at various positions within the HIV-1 env gene (as described in USP 5,866,137).

Figure 2 shows an embodiment of the invention wherein a replication defective vector that produces HIV virus like particles (VLPs) is used to induce cellular and/or humoral responses against HIV proteins. The vector, such as Toti-VacHIV, stably integrates into the genome of the host cell, and produces HIV antigens which are processed internally in the endoplasmic reticulum (ER) and expressed via the MHC class I pathway for stimulation of cellular (CD8 or cytotoxic) immune response. Additionally, proteins are produced by the integrated vector to allow formation of VLPs (via "budding" for example) that are not capable of propagating the vector.

The VLPs remain capable, however, of being taken up by cells, such as antigen presenting cells (APCs), and processed by cellular processes therein, including those that contribute to the generation of an immune response. A non-limiting example of this is shown in Figure 2, wherein an APC presents antigens from the VLP via the MHC class II pathway for stimulation of the humoral response.

Figure 3 shows the overall design of a possible embodiment of a Toti-VacHIV vector of the invention. The vector is designed to present several HIV epitopes (denoted by "E") after being (stably) introduced into a target cell. Epitopes are separated by splice sites (splice donor or "SD" and splice acceptor or "SA" sites) derived from HIV. The sites shown are "SD1", "SD2", "SA1" and "SA2". The mRNA is driven off of a constitutively expressed promoter, in this case the CMV promoter. Other promoters may be used in the practice of the invention. Non-limiting examples include the Tk promoter, the EF-α promoter, and the PGK promoter.

To inhibit or prevent propagation of the vector after administration, elements for RNA packaging (Ψ), nuclear import (central polypurine tract or cPPT), and replication (primer binding site or PBS) have been removed. It would be obvious to the skilled person, however, that 1) other deletions or mutations may be made to inhibit or prevent propagation of the vector (such as those defective in the trans-activated responsive (TAR) region, the gag carboxy-terminal CysHis box (or "zinc knuckle") region, and polypurine tract within the nef region); and 2) the three exemplary mutations may be used singly, in pairs, or in combination with other deletions or mutations to inhibit or prevent vector propagation.

Other features of the vector include sizable deletions in all epitope peptides or antigens; expression of epitopes or antigens based on their native configuration in the HIV viral genome; the gag-pol (GP) epitope is defective in both reverse transcriptase (RT) and integrase (IN) activities but able to assemble VLPs capable of "budding" from a cell; the vif-vpr region retains conserved epitopes; the tat region contains a deletion of the essential, but not known to be immunogenic, R domain for interactions with the trans-activated responsive (TAR) region; the env region contains a deletion of the cleavage site between gp120 and gp41; the nef region contains deletions in RR (double arginine), Myr (myristylation site), NBP-1 (Nef Binding Protein-1), and β-COP (β coatomer protein ) binding sites; and Rev is the only functional viral protein in this embodiment. It should be noted that many of the above changes also contribute to the generation of a replication defective vector. Other preferred embodiments have alterations (mutations and/or deletions) in all or part of the tat, vpr and/or nef regions that result in the reduction or absence of Tat, Vpr and/or Nef protein function. Particularly preferred in the practice of the invention are embodiments containing inactivating deletions of all or part of the cPPT and/or PPT. Some embodiments of this aspect of the invention do not include deficiencies in the PBS or RNA packaging sequences.

Of course replication defective vectors of the invention may contain other sequence alterations as well as the ability to express other proteins, antigens and epitopes. For example, proteins from other viruses or pathogenic microbial agents may be expressed by sequences encoding them and placed into the vectors of the invention. In one non-limiting example, this may be by the replacement strategy described above for conditionally replicating vectors.

The embodiment shown in Figure 3, and other analogous vectors based on the instant disclosure, includes, but optionally may omit, the 5' and/or 3' LTRs. Nevertheless, each of such vectors is a complete replication defective viral vector capable of presenting many epitopes concurrently. As in the case shown in Figure 2, the epitopes/antigens may be presented via MHC I (via endogenous epitope expression in cells containing the vector) and via MHC II (via VLPs released from cells containing the vector). The epitopes are expected to be in, or closely approximate, their native conformations.

A replication deficient vector of the invention may be introduced into susceptible cells, such as, but not limited to, HeLa, HeLa-tat, COS, 293, CHO, BHK, CEMx174, SupT1, Vero cells, 3T3, D17, yeast, bacteria, or primary cells *in vivo* or *ex vivo* (particularly of hematopoietic origin) capable of supporting VLP production after transfection with the vector. The introduction of the vector may be by any known means and may be transient or permanent to result in VLP expression. The VLPs may be isolated from culture supernatant by, as non-limiting examples, pelleting, sucrose gradient purification, or column chromatography. Alternatively, the vectors may be introduced into cells of a subject or patient under *ex vivo* conditions and thereafter returned to the subject or patient. The cells may be confirmed for their ability to produce VLPs before their returned or simply returned to produce VLPs *in vivo.*
Where the vector is present in a cell, however, it may be considered a replication deficient, "proviral" form even though it is replication deficient. Another embodiment of the invention that may be used is where a cell containing the vector and expressing VLPs (i.e. "VLP producer cell") is introduced into a subject or patient to generate VLPS *in vivo*. This differs from the *ex vivo* approach in that the cell need not be necessarily from the subject or patient, in which case there may be an immune response against those cells. This can be alleviated somewhat by the use of cells that constitute an allograft as opposed to a xenograft, although the appropriate use of immunosuppressive agents may be required with the use of any VLP producer cell heterologous to the subject into which they are introduced.

In an alternative embodiment of the invention, the VLPs produced by the above *in vitro* or *ex vivo* methods may be introduced into a subject or patient as therapy, thereby obviating the need for the vector to be present *in vivo*. The use of cells from the subject or patient to be treated is particularly advantageous in that the resultant VLPs may be utilized with a minimum of (or without) issues of rejection. The cells may be maintained *ex vivo* in culture to produce VLPs for extended periods via techniques known in the art.

Figure 4 shows a representation of a protocol using a replication defective vector mediated method of the invention. The embodiment begins with immunization using Toti-VacHIV DNA. The DNA may be delivered and taken up by cells of a subject by any appropriate means, including cases where the DNA is to be stably integrated into the cell's genome or maintained episomally. Expression of the epitopes by the cells result in presentation thereof in combination in an MHC I context to generate cellular based immune responses. The cells will also be able to express proteins necessary for VLP production followed by their assembly and release as VLPs into the extracellular environment or via direct cell to cell mediated transfer. Uptake of the VLPs by antigen presenting cells results in their presentation in an MHC II context to generate humoral based immune responses.

After an appropriate time, the subject would be boosted at least once with a combination of Toti-VacHIV DNA and VLPs (optionally produced by said DNA). This boost augments both the initial cellular and humoral immune responses. The induction or presence of a cellular or humoral response can be assayed at any point based on a chromium release assay for CTL activity and assays for neutralizing antibodies as known to the skilled person.

Alternatively, and not illustrated in Figure 4, subjects may be boosted separately, or in conjunction with Toti-VacHIV, in combination with another vector disclosed herein or with another vaccine as known in to the skilled person.

Any of the vectors disclosed herein may be alternatively used to express a multi-antigen construct comprising epitopes identified as of particular interest for the generation of an immune response. The multi-antigen construct preferably includes antigens or epitopes, preferably dominant determinants, which induce both cellular and humoral responses. Such antigens and epitopes may be identified by use of the vectors of the invention, which can be applied toward presenting various antigens and epitopes singly or in combination in various formats in animal models for the generation of an immune response. Those determinants found to elicit strong cellular and/or humoral responses can be selected and used in the preparation of multi-antigen constructs for expression via the vectors of the invention.

Prior to introduction into a host, a vector or construct of the present invention can be formulated into various compositions to facilitate their use in therapeutic and prophylactic treatment methods. In particular, the vectors and constructs can be made into a pharmaceutical composition by combination with appropriate pharmaceutically acceptable carriers or diluents, and can be formulated to be appropriate for either human or veterinary applications. Additionally, formulations for delayed release or release over time are also provided.

Thus, a composition for use in the method of the present invention can comprise one or more of the aforementioned vectors or constructs, preferably in combination with a pharmaceutically acceptable carrier. Pharmaceutically acceptable carriers are well-known to those skilled in the art, as are suitable methods of administration for generation of an immune response. The choice of carrier will be determined, in part, by the particular vector or construct, as well as by the particular method used to administer the composition. The skilled person will also appreciate that various routes of administering a composition are available, and, although more than one route can be used for administration, a particular route can provide a more immediate and more effective reaction than another route. Accordingly, there are a wide variety of suitable formulations of the composition of the present invention.

A composition comprised of a vector or construct of the present invention, alone or in combination with other antiviral compounds, can be made into a formulation suitable for direct administration to a subject or administration to a cell of a subject *ex vivo*. Such a formulation can include aqueous and nonaqueous, isotonic (or iso-osmotic) sterile injection solutions, which can contain antioxidants, buffers, bacteriostats, and solutes that render the formulation isotonic with the blood of the intended recipient, and aqueous and nonaqueous sterile suspensions that can include suspending agents, solubilizers, thickening agents, stabilizers, and preservatives. The formulations can be presented in unit dose or multidose sealed containers, such as ampules and vials, and can be stored in a freeze-dried (lyophilized) condition requiring only the addition of the sterile liquid carrier, for example, water, for injections, immediately prior to use. Extemporaneously injectable solutions and suspensions can be prepared from sterile powders, granules, and tablets, as described herein.

The vector can be stored in any suitable solution, buffer or lyophilizable form, if desired. A preferred storage buffer is Dulbecco's Phosphate Buffered Saline; Dulbecco's Phosphate Buffered Saline mixed with a 1-50% solution of trehalose in water (1:1), preferably a 10% solution of trehalose in water (1:1), such that the final concentration is 5% trehalose; Dulbecco's Phosphate Buffered Saline mixed with a 1-50% solution of glucose in water (1:1), preferably a 10% solution of glucose in water (1:1), such that the final glucose concentration is 5%; 20mM HEPES-buffered saline mixed with 1-50% solution of trehalose in water (1:1), preferably a 10% solution of trehalose in water (1:1), such that the final trehalose concentration is 5%; or; Dulbecco's Phosphate Buffered Saline mixed with a 1-50% solution of mannitol in water (1:1), preferably a 5% solution of mannitol in water (1:1), such that the final mannitol concentration is 2.5%.

A formulation suitable for oral administration can consist of liquid solutions, such as an effective amount of the compound dissolved in diluents, such as water, saline, or fruit juice; capsules, sachets or tablets, each containing a predetermined amount of the active ingredient, as solid or granules; solutions or suspensions in an aqueous liquid; and oil-in-water emulsions or water-in-oil emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible carriers.

A formulation suitable for oral administration can include lozenge forms, that can comprise the active ingredient in a flavor, usually sucrose and acacia or tragacanth; pastilles comprising the active ingredient in an inert base, such as gelatin and glycerin, or sucrose and acacia; and mouthwashes comprising the active ingredient in a suitable liquid carrier; as well as creams, emulsions, gels, and the like containing, in addition to the active ingredient, such carriers as are known in the art.

An aerosol formulation suitable for administration via inhalation also can be made. The aerosol formulation can be placed into a pressurized acceptable propellant, such as dichlorodifluoromethane, propane, nitrogen, and the like.

A formulation suitable for topical application can be in the form of creams, ointments, or lotions.

The dose administered to an animal, particularly a human, in the context of the present invention should be sufficient to effect a (protective) immune response in the infected individual over a reasonable time frame. The dose will be determined by the potency of the particular vector or construct employed, the severity of the disease state, as well as the body weight and age of the infected individual. The size of the dose also will be determined by the existence of any adverse side effects found to accompany the use of the particular vector or construct employed. It is always desirable, whenever possible, to keep adverse side effects to a minimum.

The dosage can be in unit dosage form. The term "unit dosage form" as used herein refers to physically discrete units suitable as unitary dosages for human and animal subjects, each unit containing a predetermined quantity of a vector or construct, alone or in combination with other antiviral agents, calculated in an amount sufficient to produce the desired effect in association with a pharmaceutically acceptable diluent, carrier, or vehicle. The specifications for the unit dosage forms of the present invention depend on the particular compound or compounds employed and the effect to be achieved, as well as the pharmacodynamics associated with each compound in the host. The dose administered should be an "effective amount" or an amount necessary to achieve an "effective level" in the individual patient.

Since the "effective level" is used as the preferred endpoint for dosing, the actual dose and schedule can vary, depending on individual differences in pharmacokinetics, drug distribution, and metabolism. The "effective level" can be defined, for example, as the blood or tissue level desired in the patient that corresponds to a concentration of one or more vectors or constructs according to the invention, which produces the desired level of immune response or protective vaccinated state, in an assay predictive for clinical efficacy. The "effective level" for use according to the present invention also can vary when the compositions of the present invention are used in combination with zidovudine or other known antiviral compounds or combinations thereof.

The skilled person can easily determine the appropriate dose, schedule, and method of administration for the exact formulation of the composition being used, in order to achieve the desired "effective level" in the individual patient. One skilled in the art also can readily determine and use an appropriate indicator of the "effective level" of the agents of the present invention by a direct (e.g., analytical chemical analysis) or indirect (e.g., with surrogate indicators of viral infection, such as p24 or reverse transcriptase for treatment of AIDS or AIDS-like disease) analysis of appropriate patient samples (e.g., blood and/or tissues).

Further, with respect to determining the effective level in a patient for treatment of AIDS or AIDS-like disease, in particular, suitable animal models are available and have been widely implemented for evaluating the *in vivo* efficacy of various immunogens against HIV. Similar models for other viruses and infectious agents are also known to the skilled person. The models include mice, monkeys and cats. Even though some of these animals are not naturally susceptible to HIV disease, chimeric mice models (e.g., SCID, bg/nu/xid, NOD/SCID, SCID-hu, immunocompetent SCID-hu, bone marrow-ablated BALB/c) reconstituted with human peripheral blood mononuclear cells (PBMCs), lymph nodes, fetal liver/thymus or other tissues can be infected with vector or HIV, and employed as models for HIV pathogenesis and gene therapy. Similarly, the simian immune deficiency virus (SIV)/monkey model can be employed, as can the feline immune deficiency virus (FIV)/cat model.

Further, with respect to determining the effective level in a patient for treatment of AIDS or AIDS-like disease, in particular, suitable animal models are available and have been widely implemented for evaluating the *in vivo* efficacy of various immunogens against HIV. Similar models for other viruses and infectious agents are also known to the skilled person. These models can also be used to determine the safety of a vector for the purposes of validation of the vector system for clinical trials. An important application is the use of these animal models for biodistribution studies. Transduced cells, preferably but not limited to human cells, containing a vector are injected into a non-human animal model and the extent of distribution of the vector is determined by the presence of vector genetic material in animal tissue. The absence of vector genetic material in animal tissue would mean that the vector does not autonomously replicate and spread to other cells and thus may be used in accord with the present invention. The presence of vector in other cells would indicate that the vector was able to propagate beyond the original cells. However, in the instance that the vectors autonomously replicate, they can be evaluated according to other criteria for safety, such as, but not limited to, the lack of replication in certain tissues or the level of replication in the animal. The presence or absence of the vector could be determined by PCR, or by FACS analysis if the tested vector expresses a marker gene that can be visualized by FACS, but is not limited to such means of detection.

Generally, an amount of vector sufficient to achieve a tissue concentration of the administered vector or construct of from about 5 µg/kg to about 300 mg/kg of body weight is preferred, especially of from about 10 µg/kg to about 200 mg/kg of body weight. The number of doses will vary depending on the means of delivery and the particular vector administered.

In the treatment of some virally infected individuals, it can be desirable to utilize a "mega-dosing" regimen, wherein a large dose of a vector is administered, time is allowed for the agent to act, and then a suitable reagent is administered to the individual to inactivate the active agent. In the method of the present invention, the treatment (i.e., the administration of conditionally replicating or replication deficient constructs) is necessarily limited.

The pharmaceutical composition can be in the form of a medicament containing other pharmaceuticals, in conjunction with a vector or construct according to the invention, when used to therapeutically treat AIDS. These other pharmaceuticals can be used in their traditional fashion (i.e., as agents to treat disease). In particular, it is contemplated that an antiretroviral agent be employed, such as, preferably, zidovudine. Further representative examples of these additional pharmaceuticals that can be used in addition to those previously described, include antiviral compounds, immunomodulators, immunostimulants, antibiotics, and other agents and treatment regimes (including those recognized as alternative medicine) that can be employed to treat AIDS. Antiviral compounds include, but are not limited to, ddI, ddC, gancylclovir, fluorinated dideoxynucleotides, nonnucleoside analog compounds such as nevirapine (Shih et al., PNAS, 88, 9878-9882 (1991)), TIBO derivatives such as R82913 (White et al., Antiviral Research, 16,257-266 (1991)), BI-RJ-70 (Shih et al., Am. J. Med., 90(Suppl. 4A), 8S-17S (1991)) and the agents and regimens known to the skilled person as described above. Immunomodulators and immunostimulants include, but are not limited to, various interleukins, CD4, cytokines, antibody preparations, blood transfusions, and cell transfusions. Antibiotics include, but are not limited to, antifungal agents, antibacterial agents, and anti-Pneumocystis carinii agents.

Administration of the virus-inhibiting compound with other anti-retroviral agents and particularly with known RT inhibitors, such as ddC, zidovudine, ddI, ddA, or other inhibitors that act against other HIV proteins, such as anti-TAT agents, will generally inhibit most or all replicative stages of the viral life cycle. The dosages of ddC and zidovudine used in AIDS or ARC patients have been published. A virustatic range of ddC is generally between 0.05 µM to 1.0 µM. A range of about 0.005-0.25 mg/kg body weight is virustatic in most patients. The dose ranges for oral administration are somewhat broader, for example 0.001 to 0.25 mg/kg given in one or more doses at intervals of 2, 4, 6, 8, and 12; etc., hr. Preferably, 0.01 mg/kg body weight ddC is given every 8 hr. When given in combined therapy, the other antiviral compound, for example, can be given at the same time as a vector according to the invention, or the dosing can be staggered as desired. The vector also can be combined in a composition. Doses of each can be less, when used in combination, than when either is used alone.

Also provided by the invention are kits comprising components such as the vectors of the invention for use in the practice of the methods disclosed herein, where such kits may comprise containers, each with one or more of the various reagents (typically in concentrated form) utilized in the methods, including, for example, buffers and other reagents as necessary. A label or indicator describing, or a set of instructions for use of, kit components in a method of the present invention, will also be typically included, where the instructions may be associated with a package insert and/or the packaging of the kit or the components thereof.

The vector-based strategies of the present invention are expected to be particularly beneficial, and more effective, against the elusive nature of certain pathogens, such as HIV. However, the invention may be easily applied to other infectious diseases with equal effectiveness. As a non-limiting example, one may consider the recent SARS threat to the human population. Once the SARS virus had been sequenced and identified, the genetic elements of the virus could be easily engineered into a vector of the instant invention, such as Toti-Vac, conditionally replicating vectors, a multiple antigen-expressing vector, or a combination thereof. The advantage provided by the present invention is the production of a vaccine capable of reliably eliciting a robust cellular and humoral response to the antigens presented thereby, without requiring extensive research and development with each emerging disease.

The following example is put forth so as to provide those of ordinary skill in the art with a complete disclosure and description of how to make and use the present invention, and is not intended to limit the scope of what the inventors regard as their invention nor are they intended to represent that the experiments below are all and only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric.

### Example

### Propagation of Vaccine HIV-based Vectors in Cell Culture

The ability of conditionally replicating HIV based vectors to complement each other in culture and propagate for a limited period of time was investigated. The first vector was an HIV-based vector containing all proteins necessary for replication, but lacking an envelope protein. The second vector expressed the envelope protein from vesicular stomatitis virus (VSV-G). Plasmids were cotransfected into 12 x 10⁶ cells 293F cells on a 1500 mm dish at 25 µg and 20 µg respectively. 24 hours post transfection, the supernatant was collected, aliquoted, and stored at -80 °C until use. Supernatant was diluted 5, 10, 50, 100, and 1000-fold in media, and added to 1 x 10⁶ HeLa-tat cells in duplicate in 6 well plates. The use of both vector constructs at increasing dilutions was used to show that co-infection in cells, as represented in Figure 1, leads to production of progeny vectors capable of infecting other cells. Such progeny vectors would be contribute to eliciting an immune response *in vivo*.

Virus propagation was measured by p24 ELISA (ABI Labs, Kensington MD) on cell supernatants as a measure of virion production. A dose-dependent level of virion production was observed, and eventual diminution of complementation occurred (Figure 5). Thus as expected the vectors replicated for a period of time before ending their replication cycles with cells that contain one of the two vector constructs. This is depicted in Figure 1 and provides an added advantage, or important safety feature, to the instant invention.

The termination of vector propagation indicates that regardless of the level of immune response that is generated, the vectors do not continue to replicate. Thus the vectors do not continue their spread after either generation of a protective immune response (immunity) or not. The generation of a protective immune response (or successful vaccination) can be tested by known assays, including but not limited to *in vitro* assays for cellular and antibodies as well as animal models of disease, such as simians for therapies targeting HIV.

Following the termination of vector propagation, and as deemed necessary by the skilled person, vector propagation may be re-activated by the repeat use of the vector combination. Alternatively, reactivation may be by the use of the individual vectors, optionally in virion form to superinfect cells that contain one or the other of the vectors originally used. As a non-limiting example, and where the first vector has all necessary components except a viral envelope protein which is provided by a second vector, the second vector capable of expressing the envelope protein may be packaged into viral particles and then introduced into cells of a subject in whom propagation of the two vectors has stopped. Infection of cells containing only the first vector by viral particles containing the second vector would reactivate propagation of the two vectors to further induce, or boost, the immune response. Of course this reactivation would again be limited in the extent of propagation in a manner analogous to that discussed and observed above.

In another embodiment of the invention, and to address situations wherein an immune response against vector elements, such as those found on a virion particle, has been generated in the treated subject, the use of a different virion particle may be used. Thus the above non-limiting example concerning a second vector used to reactivate or "boost" vector propagation may be modified such that the second vector is packaged into a viral particle displaying different antigens. For example, if the second vector was originally used via packaging in a particle displaying antigens A, B, and C, for reactivation, the same vector may be packaged into a particle displaying antigens D, E, and F to avoid the immune response in the subject.

All references cited herein, including patents, patent applications, and publications, are hereby incorporated by reference in their entireties, whether previously specifically incorporated or not

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the invention following, in general, the principles of the invention and including such departures from the present disclosure as come within known or customary practice within the art to which the invention pertains and as may be applied to the essential features hereinbefore set forth. Additional embodiments of the invention are listed below.
1. A method of inducing an immune response in a subject, said method comprising administering a system of two or more conditionally replicating lentiviral vectors to a cell of said subject, wherein each of said two or more vectors replicates only in the presence of the other vectors in the system, and said system of vectors expresses one or more antigens to which an immune response is desired in said subject.
2. The method of embodiment 1, wherein at least one of said vectors contains a genetic antiviral agent against one or more other vectors in said system.
3. The method of embodiment 1, wherein said system comprises two lentiviral vectors.
4. The method of embodiment 2, wherein only one of said vectors comprises an env encoding sequence.
5. The method of any one of embodiments 1 to 4, wherein said administering occurs *ex vivo*.
6. The method of any one of embodiments 1 to 4, wherein said immune response is a cellular in nature and comprises the potentiation of CTL and/or CD4+ cells.
7. The method of embodiment 5, wherein said immune response is a cellular in nature and comprises the potentiation of CTL and/or CD4+ cells.
8. The method of embodiments 1 to 4, wherein said immune response is protective against a virus or microorganism expressing one or more antigens expressed by said vectors.
9. The method of embodiment 5, wherein said immune response is protective against a virus or microorganism expressing one or more antigens expressed by said vectors.
10. The method of any one of embodiments 1 to 4, wherein said one or more antigens is one or more HIV antigens.
11. The method of embodiment 5, wherein said one or more antigens is one or more HIV antigens.
12. The method of inducing an immune response in a subject, said method comprising administering a system of two or more lentiviral vectors to a cell of said subject, wherein said system of vectors expresses the proteins needed to form a virus like-particle, and at least one of said vectors cannot be packaged into said particle.
13. A replication deficient lentiviral vector comprising a deletion of all or part of the central polypurine tract and a heterologous promoter capable of directing expression of viral proteins encoded by said vector.
14. A method of inducing an immune response in a subject, said method comprising administering a replication deficient lentiviral vector according to embodiment 13 to a cell of said subject,
   wherein said vector expresses the proteins needed to form a virus like-particle.
15. The method of any one of embodiments 1 to 4, 12 and 14, wherein said one or more antigens is expressed by a multi-antigen encoding construct which results in the expression of multiple viral epitopes as a single polypeptide.
16. The method of embodiment 5, wherein said one or more antigens is expressed by a multi-antigen encoding construct which results in the expression of multiple viral epitopes as a single polypeptide.
17. The method of embodiment 6, wherein said one or more antigens is expressed by a multi-antigen encoding construct which results in the expression of multiple viral epitopes as a single polypeptide.
18. The method of embodiment 7, wherein said one or more antigens is expressed by a multi-antigen encoding construct which results in the expression of multiple viral epitopes as a single polypeptide.
19. The method of embodiment 8, wherein said one or more antigens is expressed by a multi-antigen encoding construct which results in the expression of multiple viral epitopes as a single polypeptide.
20. The method of embodiment 9, wherein said one or more antigens is expressed by a multi-antigen encoding construct which results in the expression of multiple viral epitopes as a single polypeptide.
21. The method of embodiment 10, wherein said one or more antigens is expressed by a multi-antigen encoding construct which results in the expression of multiple viral epitopes as a single polypeptide.
22. The method of embodiment 11, wherein said one or more antigens is expressed by a multi-antigen encoding construct which results in the expression of multiple viral epitopes as a single polypeptide.
23. The method of any one of embodiments 1 to 4, 12 and 14, wherein said vector comprises a sequence encoding a non-lentiviral antigen.
24. The method of embodiment 5, wherein said vector comprises a sequence encoding a non-lentiviral antigen.
25. The method of embodiment 6, wherein said vector comprises a sequence encoding a non-lentiviral antigen.
26. The method of embodiment 7, wherein said vector comprises a sequence encoding a non-lentiviral antigen.
27. The method of embodiment 8, wherein said vector comprises a sequence encoding a non-lentiviral antigen.
28. The method of embodiment 9, wherein said vector comprises a sequence encoding a non-lentiviral antigen.
29. The method of embodiment 10, wherein said vector comprises a sequence encoding a non-lentiviral antigen.
30. The method of embodiment 11, wherein said vector comprises a sequence encoding a non-lentiviral antigen.
31. The method of any one of embodiments 1 to 4, 12 and 14, wherein said vector comprises a sequence encoding a non-lentiviral antigen, and wherein said non-lentiviral antigen is from a virus selected from the group consisting of retroviruses, togaviruses, rhabdoviruses, paramyxoviruses, herpesviruses, orthomyxoviruses, and coronaviruses.
32. The method of embodiment 24, wherein said non-lentiviral antigen is from a virus selected from the group consisting of retroviruses, togaviruses, rhabdoviruses, paramyxoviruses, herpesviruses, orthomyxoviruses, and coronaviruses.
33. The method of embodiment 25, wherein said non-lentiviral antigen is from a virus selected from the group consisting of retroviruses, togaviruses, rhabdoviruses, paramyxoviruses, herpesviruses, orthomyxoviruses, and coronaviruses.
34. The method of embodiment 26, wherein said non-lentiviral antigen is from a virus selected from the group consisting of retroviruses, togaviruses, rhabdoviruses, paramyxoviruses, herpesviruses, orthomyxoviruses, and coronaviruses.
35. The method of embodiment 27, wherein said non-lentiviral antigen is from a virus selected from the group consisting of retroviruses, togaviruses, rhabdoviruses, paramyxoviruses, herpesviruses, orthomyxoviruses, and coronaviruses.
36. The method of embodiment 28, wherein said non-lentiviral antigen is from a virus selected from the group consisting of retroviruses, togaviruses, rhabdoviruses, paramyxoviruses, herpesviruses, orthomyxoviruses, and coronaviruses.
37. The method of embodiment 29, wherein said non-lentiviral antigen is from a virus selected from the group consisting of retroviruses, togaviruses, rhabdoviruses, paramyxoviruses, herpesviruses, orthomyxoviruses, and coronaviruses.
38. The method of embodiment 30, wherein said non-lentiviral antigen is from a virus selected from the group consisting of retroviruses, togaviruses, rhabdoviruses, paramyxoviruses, herpesviruses, orthomyxoviruses, and coronaviruses.

## Claims

1. A lentiviral vector comprising:
(a) a 5' long terminal repeat (LTR) and a 3'LTR;
(b) a nucleic acid sequence encoding an antigenic epitope; and
(c) a nucleic acid sequence encoding a rev response element (RRE).

2. The vector of claim 1, further comprising a packaging sequence in the 5' leader and gag encoding sequences of the lentivirus.

3. The vector of claim 1, wherein the epitope is from: gag-pol, vif, vpr, or env; derived from the unspliced or partially spliced messenger RNA of gag-pol, vif, vpr, or env; tat, rev, or nef; or derived from the multiply spliced messenger RNA of tat, rev, or nef.

4. The vector of claim 1, further comprising a nucleic acid sequence encoding a structural or non-structural viral protein.

5. The vector of claim 4, wherein the protein is rev.
